# EUROPÄISCHE PATENTSCHRIFT

(11) **EP 0 900 094 B1**
(45) Veröffentlichungstag und Bekanntmachung des Hinweises auf die Patenterteilung: **24.09.2003**
(21) Anmeldenummer: 98904114.0
(22) Anmeldetag: 22.01.1998
(51) Int. Cl.: A61M 1/36

(54) **VORRICHTUNG ZUR BESTIMMUNG DER REZIRKULATION WÄHREND EINER EXTRAKORPORALEN BLUTBEHANDLUNG**
DEVICE FOR MONITORING RECIRCULATION DURING AN EXTRACORPOREAL BLOOD TREATMENT
DISPOSITIF DE SURVEILLANCE DE LA RECIRCULATION PENDANT UN TRAITEMENT EXTRACORPOREL DU SANG

(30) Priorität: 24.01.1997 DE 19702441
(43) Veröffentlichungstag der Anmeldung: 10.03.1999
(73) Patentinhaber: Fresenius Medical Care Deutschland GmbH, 61352 Bad Homburg v.d.H. (DE)
(72) Erfinder: GOLDAU, Rainer, D-97222 Rimpar (DE)
(74) Vertreter: Luderschmidt, Schüler & Partner GbR
(86) Internationale Anmeldenummer: EP9800325
(87) Internationale Veröffentlichungsnummer: WO98032477

(56) Entgegenhaltungen:
- EP-A- 0 272 414
- EP-A- 0 693 297
- WO-A-96/08305
- DE-A- 4 024 434
- US-A- 5 510 717
- US-A- 5 588 959

## Beschreibung

Die Erfindung betrifft eine Vorrichtung zur Bestimmung der Rezirkulation während einer extrakorporalen Blutbehandlung in Verbindung mit einer Blutbehandlungsvorrichtung, bei der das zu behandelnde Blut in einem extrakorporalen Kreislauf die Blutkammer eines durch eine semipermeable Membran in die Blutkammer und eine Dialysierflüssigkeitskammer unterteilten Dialysators durchströmt und Dialysierflüssigkeit in einem Dialysierflüssigkeitsweg die Dialysierflüssigkeitskammer des Dialysators durchströmt.

Bei Verfahren der chronischen Blutreinigungstherapie, wie Hämodialyse, Hämofiltration und Hämodiafiltration, wird Blut über einen extrakorporalen Kreislauf geleitet. Als Zugang zum Blutgefäßsystem wird häufig operativ eine arteriovenöse Fistel angelegt. Ebenso ist der Einsatz eines Implantats möglich. Wenn nachfolgend von dem Begriff "Fistel" die Rede ist, wird darunter jede Art der Verbindung zwischen einer Vene und einer Arterie des Patienten verstanden.

Das durch die Fistel flie8ende Blut wird nur während der eigentlichen Dialysebehandlung benutzt. Im dialysefreien Zeitraum entspricht der Blutflu8 in der Fistel (Q_{FI}) einem funktionellen Links/Rechts-Shunt, bei dem ein Anteil des arteriellen Blutes aus dem Herzminutenvolumen (HMV) unter Umgehung einer peripheren Nutzung direkt dem venösen System und dem Herzen zugeführt wird. Der Fistelfluß rezirkuliert über Herz und Lungen. Der fraktionelle Anteil des Fistelflusses am Herzminutenvolumen wird als kardiopulmonäre Rezirkulation definiert. Die kardiopulmonäre Rezirkulation (CPR) hat nicht nur Auswirkungen auf die Kreislaufbelastung des Patienten insgesamt, sondern hat auch Auswirkungen auf die Effizienz der Dialyse. Da das dialysierte Blut aus dem extrakorporalen Kreislauf unter Umgehung der systemischen Kreislaufgebiete dem venösen Rückstrom aus dem großen Körperkreislauf beigemischt wird, kommt es zu einer systematischen Reduktion in der Konzentration dialysierbarer Bestandteile im arteriellen Blut (D. Schneditz et al.; Cardiopulmonary recirculation during hemodialysis. Kidney Int. 42:1450-1456, 1992).

Für die Funktionsfähigkeit der Fistel ist deren Perfusion von Bedeutung. Sinkt der Fistelfluß unter einen kritischen Wert, dann steigt das Risiko einer Fistelthrombose mit dem möglichen Verlust des Gefäßzuganges, was in der Dialysebehandlung eine erhebliche Komplikation darstellt (W. Bay et al.: Color Doppler flow predicts PTFE graft failure, J. Am. Soc. Nephrol. 5:407 (1994)). Ist der Fistelfluß während der Hämodialysebehandlung unzureichend und kleiner als der extrakorporale Blutfluß (Q_{B}), kommt es zur lokalen Fistelrezirkulation, wobei eine Fraktion des dialysierten und mit der venösen Blutleitung zur Fistel zurückgeführten Blutes über die arterielle Blutleitung dem Dialysator wieder zugeführt wird. Die Fistelrezirkulation bewirkt eine bedeutende Verminderung der Dialyseeffizienz (F. Gotch: "Models to predict recirculation and its effect on treatment time in single-needle dialysis" First Intl. Symposium on Single-Needle Dialysis, Hrsg.: S. Rignoir. R. Vanholder und P. Invanovich, Cleveland, ISAO Press, 1984, Seite 305 ff.). Die Messung der Qualität des Gefäßzuganges ist damit ein wichtiges Mittel zur Qualitätssicherung bei der Dialysebehandlung.

Aufgrund ihrer klinischen Bedeutung sind eine Reihe von Verfahren zur Messung der Rezirkulation bekannt. Allen gemeinsam ist die Messung einer physikalischen oder chemischen Kenngröße des Blutes, die im venösen Zweig des extrakorporalen Kreislaufs verändert wird. Die physikalische oder chemische Kenngröße des Blutes kann durch eine manuelle Injektion eines Indikators oder auch mittelbar über die Dialysataufbereitungseinheit geändert werden.

Aus EDTNA-ERCA Journal 19,6 (1993) ist ein als Thermodilution bezeichnetes Verfahren zur Rezirkulationsmessung bekannt. Bei dem bekannten Verfahren wird ein kurzzeitiger Temperaturabfall im Dialysierflüssigkeitskreislauf infiziert, der sich auf den venösen Zweig des extrakorporalen Kreislaufs überträgt und zu einem nachweisbaren Temperatursprung im arteriellen Zweig des extrakorporalen Kreislaufs dann führt, wenn eine Rezirkulation auftritt.

In dem Aufsatz von Niels A. Lassen, Ole Henriksen, Per Sejrsen in Handbook of Physiology, The Cardiovascular System, Vol. 3, Peripheral Circulation and Organ Blood Flow, Part I, American Physiological Society, 1983, wird die Bolusantwort eines intrakorporalen Kreislaufs auf eine Injektion und eine anschließende Messung der Konzentration näher behandelt, wobei Fragen der Signalfaltung eine wichtige Rolle spielen.

Eine bekannte Vorrichtung zur Durchführung des als Thermodilution bezeichneten Verfahrens weist einen im arteriellen Zweig und einen im venösen Zweig des extrakorporalen Kreislaufs angeordneten Temperaturfühler auf. Mit dem venösen Temperaturfühler wird der Temperatursprung aufgenommen, der auf den im Dialysierflüssigkeitskreislauf erzeugten Temperaturabfall zurückzuführen ist. Der gemessene Temperatursprung wird zeitlich integriert und nachfolgend mit dem im arteriellen Meßfühler registrierten Temperaturverlauf verglichen. Das Verhältnis der beiden Temperatur-Integrale zueinander ist ein Maß für die gesamte Effienzminderung der Dialysebehandlung durch fistel- und kardiopulmonäre Rezirkulation.

Die bekannte Vorrichtung zur Rezirkulationsmessung hat sich in der Praxis bewährt. Als nachteilig erweist sich jedoch der verhältnismäßig hohe apparative Aufwand aufgrund der im extrakorporalen Kreislauf angeordneten Temperaturfühler als auch die verbleibende Wechselwirkung des rezirkulierten Blutes mit der Temperatur des Körpers.

Aus der EP-A-693 297 ist eine Dialysemaschine bekannt, bei der zur Bestimmung der Menge der von der Vene über die Fistel zu der Arterie rezirkulierten Blutes die Na-Konzentration im Blut stromab des Dialysators variiert und die Na-Konzentration im Blut stromauf des Dialysators gemessen wird. Nachteilig ist, daß zur Änderung der Na-Konzentration im Blut ein Eingriff in den extrakorporalen Kreislauf erforderlich ist. Für die Messung der Na-Konzentration des Blutes stromab des Dialysators wird als Alternative vorgeschlagen, die Konzentration im Ultrafiltrat zu messen, das über einen Hämofilter dem extrakorporalen Kreislauf entzogen wird. Diese bekannte Vorrichtung ermöglicht das Bestimmen einer Änderung der physikalischen oder chemischen Kenngrößen des Blutes auf der Blutseite.
Keine Veränderung aber der physikalischen oder chemischen Kenngrößen der Dialysierflüssigkeit stromab des Dialysators wird hier gemessen.

Der Erfindung liegt die Aufgabe zugrunde, eine Vorrichtung zur Bestimmung der Rezirkulation während der extrakorporalen Blutbehandlung anzugeben, die einen relativ geringen apparativen Aufwand erfordert, wobei von Meßfühlern Gebrauch gemacht werden kann, die im allgemeinen ohnehin in den bekannten Blutbehandlungsvorrichtungen vorhanden sind.

Die Lösung der Aufgabe erfolgt erfindungsgemäß mit den im Patentanspruch 1 angegebenen Merkmalen.

Ein besonderer Vorteil der Vorrichtung gemäß der Erfindung liegt darin, daß zur Meßwerterfassung keine Meßfühler im arteriellen oder venösen Zweig des extrakorporalen Kreislaufs erforderlich sind. Die gesamte Sensorik ist auf die weniger anfällige Dialysatseite beschränkt, auf der entsprechende Detektoren in den bekannten Blutbehandlungsvorrichtungen im allgemeinen bereits vorhanden sind.

Bei der erfindungsgemäßen Vorrichtung wird eine physikalische oder chemische Kenngröße der Dialysierflüssigkeit im Dialysierflüssigkeitsweg stromauf des Dialysators verändert, die zu einer Änderung der physikalischen oder chemischen Kenngröße auf der Blutseite führt. Alternativ kann die Flu8rate des durch die Blutkammer des Dialysators strömenden Blutes oder die Flußrate der durch die Dialysierflüssigkeitskammer des Dialysators strömenden Dialysierflüssigkeit in einem vorgegebenen Zeitintervall verändert werden. Bei in-vitro-Messungen hat sich nämlich gezeigt, daß z.B. eine kurzzeitige deutliche Verringerung der Blutflußrate, eine gut meßbare Rückwirkung auf die Konzentration des venösen Blutes, d.h. die Blutausgangskonzentration, zur Folge hat.

Die Änderung der physikalischen oder chemischen Kenngröße auf der Blutseite führt im Falle der Fistelrezirkulation zu einer Änderung der physikalischen oder chemischen Kenngröße der Dialysierflüssigkeit stromab der Dialysierflüssigkeitskammer des Dialysators. Zur Bestimmung der Rezirkulation wird die physikalische oder chemische Kenngröße im Dialysierflüssigkeitsweg stromab des Dialysators gemessen und aus dem zeitlichen Verlauf der Änderung der physikalischen oder chemischen Kenngröße wird die Rezirkulation bestimmt.

Zur Bestimmung der Rezirkulation ist nur eine einzige Meßeinrichtung im Dialysierflüssigkeitsweg stromab des Dialysators notwendig, sofern die aufgezwungene Änderung der physikalischen oder chemischen Kenngröße im Dialysierflüssigkeitsweg ihrem zeitlichen Verlauf nach bekannt ist. Wenn dies nicht der Fall ist, werden zwei Meßeinrichtungen im Dialysierflüssigkeitsweg stromauf und stromab des Dialysators benötigt.

Als physikalische oder chemische Kenngröße wird vorteilhafterweise die Dialysierflüssigkeits-Ionen-Konzentration, z.B. die Na-Konzentration der Dialysierflüssigkeit verändert und gemessen, die durch eine Leitfähigkeitsmessung bestimmt werden kann. Dies ist insofern von Vorteil, als das rezirkulierende Blut bezüglich der Leitfähigkeit keine Wechselwirkung im Körper des Patienten erfährt und die zur Bestimmung der Leitfähigkeit erforderlichen Meßeinrichtungen in den bekannten Dialysegeräten im allgemeinen bereits vorhanden sind. Es ist aber auch möglich, als physikalische oder chemische Kenngröße beispielsweise die Dichte, die Temperatur oder den Brechungsindex zu messen, d.h., alle Größen, die im Rezirkulationsast des Körpers bezüglich ihrer Größe oder zeitlich eine andere Wechselwirkung als im Kapillarsystem erfahren und sich über den Dialysator übertragen lassen.

Die Messung kann wie folgt ablaufen. Zunächst wird ein kurzer Konzentratbolus auf die in den Dialysator fließende Dialysierflüssigkeit gegeben oder der Blutfluß wird kurzzeitig deutlich verlangsamt. Während das im venösen Zweig des extrakorporalen Kreislaufs fließende Blut in den Körper zu fließen beginnt, wird die Änderung auch an der Dialysierflüssigkeitsausgangskonzentration, d.h. der Konzentration der aus der Dialysierflüssigkeitskammer des Dialysators strömenden Dialysierflüssigkeit, erstmalig sichtbar. Nach Ablauf der Rezirkulationszeit erscheint ein Teil des Blutes wieder am Dialysator und bewirkt erneut eine Änderung der Dialysierflüssigkeitsausgangskonzentration, die gemessen und ausgewertet wird. Die zweite Änderung hängt im Gegensatz zu der ersten Änderung vom Grad der Rezirkulation ab, wobei die zweite Änderung umso stärker ausfällt, je höher die Rezirkulation ist. Im Körper des Patienten kommt es zu keiner Konzentrations- bzw. Leitfähigkeitsänderung des rezirkulierten Blutes. Nur der nichtrezirkulierte Anteil des Blutes, der das Kapillarsystem durchströmt hat, erfährt eine solche.

Mit der erfindungsgemäßen Vorrichtung kann bei Vertauschung der Gefäßzugänge über eine erzwungene Fistelrezirkulation auch der Fistelfluß bestimmt werden.
Nachfolgend wird die erfindungsgemäße Vorrichtung zur Durchführung des Verfahrens unter Bezugnahme auf die Figuren näher erläutert.

Es zeigen:

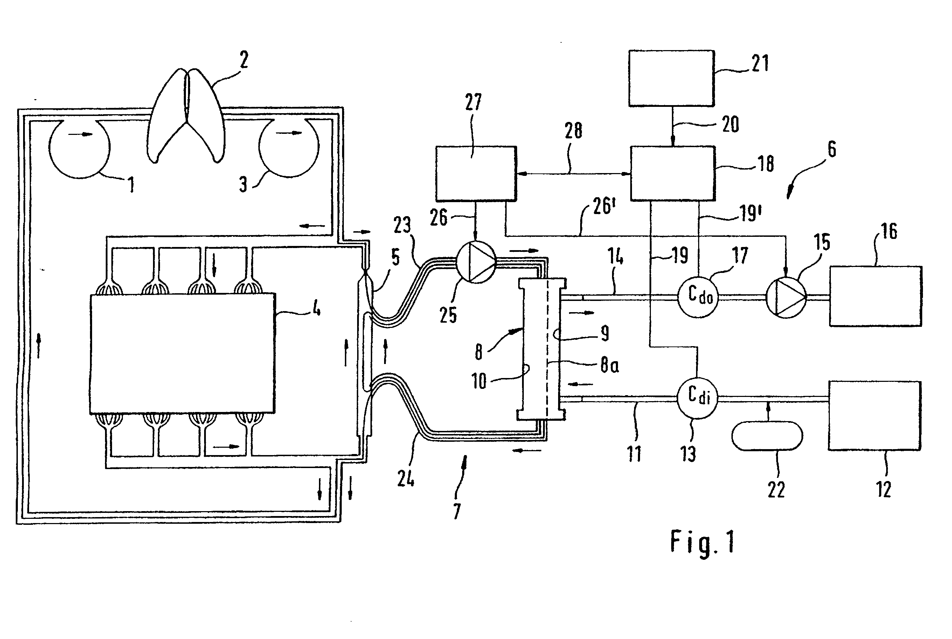

- Fig. 1: eine schematische Darstellung der erfindungsgemäßen Vorrichtung zur Bestimmung der Rezirkulation in Verbindung mit einer Dialysevorrichtung einschlie8lich des intrakorporalen Kreislaufs,
- Fig. 2a: den zeitlichen Verlauf der Dialysierflüssigkeitseingangskonzentration stromauf des Dialysators,
- Fig. 2b: den zeitlichen Verlauf der Dialysierflässigkeitsausgangskonzentration stromab des Dialysators, wobei keine Rezirkulation auftritt,
- Fig. 3: den zeitlichen Verlauf der Dialysierflηssigkeitsausgangskonzentration im Falle einer Rezirkulation, wobei der Antwortbolus deutlich vom durchlaufenden Bolus getrennt ist und
- Fig. 4: den zeitlichen Verlauf der Dialysierflüssigkeitsausgangskonzentration im Falle einer Rezirkulation noch während der Bolusgabe, wobei der durchlaufende Bolus von dem Antwortbolus überlagert wird.

Die erfindungsgemäße Vorrichtung zur Bestimmung der Rezirkulation des Blutes kann eine separate Baugruppe bilden. Sie kann aber auch Bestandteil einer Dialysevorrichtung sein, zumal einige Komponenten der erfindungsgemäßen Vorrichtung in den bekannten Dialysevorrichtungen bereits vorhanden sind. Nachfolgend wird die erfindungsgemäße Vorrichtung zusammen mit den wesentlichen Komponenten der Dialysevorrichtung einschließlich des intrakorporalen Kreislaufs beschrieben.

Der intrakorporale Kreislauf umfaßt das rechte Ventrikel 1 des Herzens, die Lunge 2, das linke Ventrikel 3 und sämtliche Kapillarsysteme 4 des Körpers in inneren Organen, Muskulatur und Haut etc.. Um einen Zugang zu dem Blutgefäßsystem zu schaffen, ist eine arteriovenöse Fistel 5 angelegt.

Die Dialysevorrichtung besteht im wesentlichen aus einem Dialysierflüssigkeitsteil 6 und einem extrakorporalen Blutkreislauf 7, zwischen denen sich ein Dialysator 8 mit einer Dialysierflüssigkeitskammer 9 und einer Blutkammer 10 befindet, die durch eine semipermeable Membran 8a voneinander getrennt sind. Die Dialysierflüssigkeitskammer 9 ist stromauf des Dialysators 8 über eine Dialysierflüssigkeitsleitung 11 mit einer Dialysierflüssigkeitsquelle 12 verbunden. In die Dialysierflüssigkeitsleitung ist eine Meßeinrichtung 13 mit einem Leitfähigkeitssensor oder einem optischen Sensor zur Bestimmung der Dialysierflüssigkeitsseingangskonzentration C_{di} geschaltet. Stromab des Dialysators 8 ist an die Dialysierflüssigkeitskammer eine weitere Leitung 14 angeschlossen, die eine Dialysierflüssigkeitspumpe 15 aufweist und die in einen Abfluß 16 führt. In den Dialysierflüssigkeitsweg stromab des Dialysators 8 ist eine Meßeinrichtung 17 mit einem Leitfähigkeitssensor oder einem optischen Sensor zur Bestimmung der Dialysierflüssigkeitsausgangskonzentration C_{do} geschaltet. Ferner ist eine Speichereinheit 18 vorgesehen, die über Datenleitungen 19, 19' die Meßwerte der Meßeinrichtungen 13, 17 empfängt und in zeitlicher Abfolge abspeichert. Die Speichereinheit 18 ist über eine Signalleitung 20 mit einer Rechen- und Auswerteinheit 21 verbunden. Mit dem Bezugszeichen 22 ist eine stromauf des Dialysators 8 angeordnete Einrichtung bezeichnet, mit der auf die in den Dialysator fließende Dialysierflüssigkeit ein Konzentratbolus aufgegeben werden kann.

Der extrakorporale Kreislauf 7 umfaßt einen arteriellen Zweig 23, der mit dem arteriellen Teil der Fistel 5 in Verbindung steht, die Blutkammer 10 des Dialysators 8 und einen venösen Zweig 24, der mit dem venösen Teil der Fistel in Verbindung steht. Im arteriellen Zweig 23 des extrakorporalen Kreislaufs 7 ist eine Blutpumpe 25 angeordnet, die über eine Steuerleitung 26 mit einer Steuereinheit 27 verbunden ist, mit der die Förderrate der Blutpumpe 25 verändert werden kann. Die Steuereinheit steht über eine weitere Steuerleitung 26' mit der Dialysierflüssigkeitspumpe 15 und über eine Signalleitung 28 mit der Rechenund Auswerteinheit 21 in Verbindung.

Nachfolgend wird die Funktion der Vorrichtung und das Prinzip der Messung im einzelnen erläutert.

Das vom linken Ventrikel 1 emittierte Blut fließt zum größten Teil in die Kapillarsysteme aller Organe, zu einem kleinen Teil in die Fistel 5. Für den Fall, daß der Blutfluß im extrakorporalen Kreislauf kleiner als der Blutfluß des in die Fistel bzw. aus der Fistel fließenden Blutes ist, fließt das Fistelblut zum einen Teil durch den extrakorporalen Kreislauf 7, zum anderen Teil durch die Fistel 5. Wenn der extrakorporale Blutfluß größer als der Fistelfluß ist, dann rezirkuliert Blut aus dem extrakorporalen Kreislauf, wobei die Fistel vom venösen zum arteriellen Anschluß durchflossen wird. Das extrakorporale Blut, das durch die Fistel fließende Blut und das aus den Kapillarsystemen stammende Blut vereinigt sich schließlich wieder im Rücklauf zum Herzen.

Das Verfahren wo die Erfindung Anwendung finden kann, beruht darauf, daß die Dialysierflüssigkeitseingangskonzentration C_{di}, z.B. die Na-Konzentration des Dialysats, stromauf des Dialysators 8 mittels der Einrichtung 22 zur Erzeugung eines Konzentratbolus kurzzeitig verändert und die Dialysierflüssigkeitseingangs- C_{di} und -ausgangskonzentration C_{do} mittels der Meßeinrichtungen 13, 17 stromauf und stromab des Dialysators gemessen werden. Alternativ kann auch die Förderrate der im extrakorporalen Kreislauf 7 angeordneten Blutpumpe 25 oder die Förderrate der Dialysierflüssigkeitspumpe 15 mittels der Steuereinrichtung 27 kurzzeitig verringert werden. Die kurzzeitige Verringerung der Blutflußrate oder der Dialysierflüssigkeitsrate führt ebenfalls zu einer Veränderung der Dialysierflüssigkeitsausgangskonzentration C_{do}. Ist die Änderung der Dialysierflüssigkeitseingangskonzentration C_{di} ihrem zeitlichen Verlauf nach bekannt, kann die Meßeinrichtung 13 stromauf des Dialysators 8 entfallen. Diese ist nur dann erforderlich, wenn ein nicht definierter Konzentratbolus aufgegeben wird.

Anstelle eines Konzentratbolus kann im Dialysierflüssigkeitsweg stromauf des Dialysators 8 auch ein Temperatursprung erzeugt werden. Bei einer derartigen Ausführungsform ist die Einrichtung 22 zum Verändern der physikalischen oder chemischen Kenngröße der Dialysierfiüssigkeit als Heizeinrichtung ausgebildet und die Meßeinrichtungen 13, 17 weisen Temperatursensoren zum Messen der Temperatur der Dialysierflüssigkeit auf.

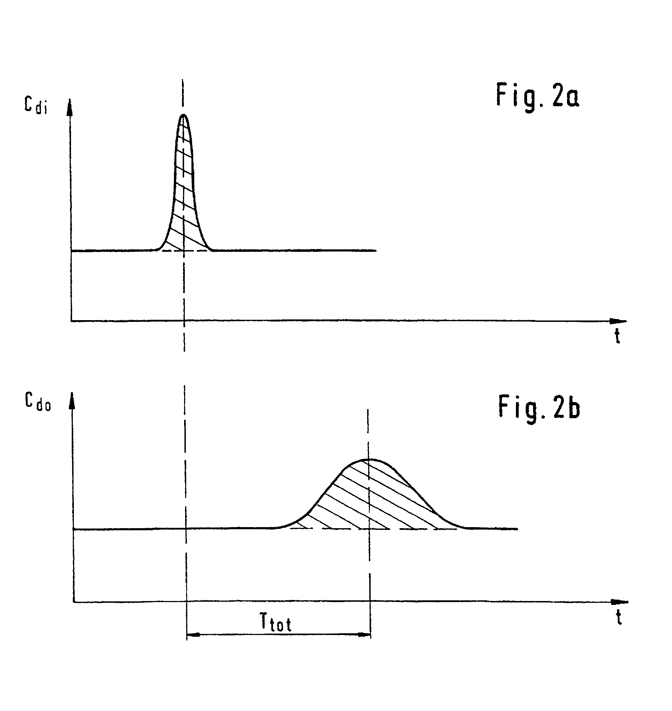

Fig. 2a zeigt den zeitlichen Verlauf der Änderung der Dialysierflüssigkeitseingangs- C_{di} und Fig. 2b den zeitlichen Verlauf der Änderung der Dialysierflüssigkeitsausgangskonzentration C_{do}, sofern eine Rezirkulation nicht auftritt. Der Konzentrationsbolus passiert die stromab des Dialysators 8 angeordnete Meßeinrichtung 17 nach der Flußzeit Tₜₒₜ.

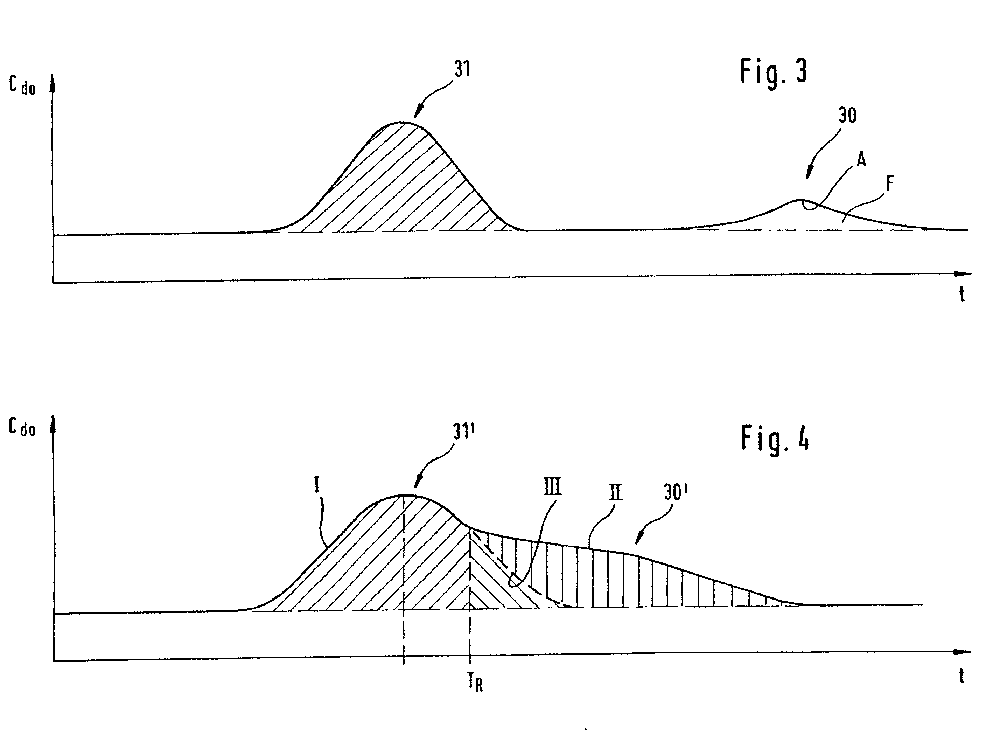

Wenn eine Rezirkulation auftritt, ist zwischen zwei Fällen zu unterscheiden, die in den Fign. 3 und 4 dargestellt sind.

Fig. 3 zeigt den Fall, daß der auf die Rezirkulation zurückzuführende Antwortbolus 30 von dem durchlaufenden Bolus 31 getrennt ist, der auf die Änderung der Konzentration der in den Dialysator 8 fließenden Dialysierflüssigkeit zurückzuführen ist. Hier ist die Fläche F oder die Amplitude A des Antwortbolus ein Matt für die Rezirkulation. Je größer die Fläche oder die Amplitude ist, desto größer ist die Rezirkulation.

Im Fall einer kurzzeitigen Rezirkulation noch während der Bolusgabe wird am Ausgang des Dialysators 8 der in Fig. 4 gezeigte Kurvenlauf gemessen. Der durchlaufende Bolus 31' wird von dem Antwortbolus 30' überlagert. Der Zeitpunkt, zu dem die Rezirkulation einsetzt, ist in Fig. 4 mit T_{R} bezeichnet. Die Rezirkulationszeit T_{R}, die für eine bestimmte Konfiguration (Förderrate, Schlauchset, Dialysator etc.), in der die Vorrichtung zur Bestimmung der Rezirkulation Verwendung findet, zuvor in Kalibrierversuchen bestimmt wurde, ist in der Rechen- und Auswerteinheit abgelegt. Für den Zeitbereich t < T_{R} kann eine Rezirkulation noch nicht stattgefunden haben (Kurve I). In diesem Zeitbereich entspricht die Funktion C_{do}(t) der Reaktion eines linearen zeitinvarianten Systems auf den Konzentratbolus am Dialysatoreingang (Fig. 2a). Zum Zeitpunkt t = T_{R} kehrt das Blut, dessen physikalische oder chemische Kenngröße verändert wurde, wieder zum Dialysator zurück und führt zu einer Veränderung der Kenngröße im Dialysierflüssigkeitsweg stromab des Dialysators, die von der Meßeinrichtung 17 erfaßt wird. Für den Zeitraum t ≥ T_{R} wird der durchlaufende Bolus dann von dem auf die Rezirkulation zurückzuführenden Antwortbolus überlagert (Kurve II).

Die Funktion C_{do}(t) umfaßt für den Fall, daß eine Rezirkulation nicht auftritt, die Kurven I und III (Fig. 4). Wenn die Rezirkulation gleich Null ist, hat die Funktion C_{do}(t) einen symmetrischen Verlauf. Daher kann die Rezirkulation durch einen Symmetrievergleich ermittelt werden.

Zur Bestimmung der Rezirkulation wird die Signalantwort (Fign. 3 und 4) auf den Konzentrationsbolus (Fig. 2a) stromab des Dialysators 8 mittels der Meßeinrichtung 17 gemessen und in der Speichereinheit 18 gespeichert. In der Rechen- und Auswerteinheit 21, die vorzugsweise als Digitalrechner ausgebildet ist, wird die den zeitlichen Verlauf der Dialysierflüssigkeitsausgangskonzentration C_{dn}(t) darstellende Funktion analysiert.

Zunächst wird in der Rechen- und Auswerteinheit 21 bei der Analyse der Funktion festgestellt, ob der in Fig. 3 oder 4 gezeigte Kurvenverlauf vorliegt.

Wird der in Fig. 3 gezeigte Kurvenverlauf festgestellt, so wird die Fläche F oder Amplitude A des Antwortbolus 30 aus den gespeicherten Funktionswerten berechnet. Die Auswert- und Recheneinheit 21 verfügt über einen Komparator, der den ermittelten Wert für die Fläche oder Amplitude mit Referenzwerten vergleicht, die für eine bestimmte Rezirkulation charakteristisch sind. Diese zuvor in Kalibrierversuchen ermittelten Referenzwerte sind in der Auswert- und Recheneinheit 21 abgelegt. Bei einer Übereinstimmung mit einem der Referenzwerte wird die entsprechende Rezirkulation dann von der Auswert- und Recheneinheit 21 ausgegeben.

Wird hingegen der in Fig. 4 gezeigte Kurvenverlauf festgestellt, kann der auf die Rezirkulation zurückzuführende Antwortbolus nicht direkt bestimmt werden. In der Auswert- und Recheneinheit wird daher zunächst die Übertragungsfunktion oder Stoßantwort, d.h. die Reaktion des Systems auf einen Diracstoß als Eingangssignal berechnet. Um die Signalübertragung nicht durch die Lösung eines Faltungsintegrals zu berechnen, werden das Eingangs- und Ausgangssignal in der Rechen- und Auswerteinheit einer Laplace-Transformation unterzogen, so daß sich die Übertragung in bekannter Weise durch ein algebraisches Produkt darstellen läßt. Durch Rücktransformation in den Zeitbereich wird dann aus der Übertragungsfunktion die Stoßantwort berechnet.

Nachdem die Stoßantwort ermittelt ist, wird aus dem seinem zeitlichen Verlauf nach bekannten Konzentrationsbolus am Dialysatoreingang (Fig. 2a) und der Stoßantwort die in Fig. 4 in gestrichelter Linie dargestellte Funktion berechnet, die den zeitlichen Verlauf der Dialysierflüssigkeitsausgangskonzentration C_{do} für den Fall angibt, daß eine Rezirkulation nicht auftritt (Kurve III). Anschließend wird die hypothetische Funktion (Kurve III) von der gemessenen und abgespeicherten Funktion (Kurve II) subtrahiert, wodurch der auf die Rezirkulation zurückzuführende Antwortbolus 30' in der Auswert- und Recheneinheit 21 ermittelt wird. Damit ist der Fall, daß sich der Antwortbolus 30' mit dem durchlaufenden Bolus 31' überlagert, auf den Fall zurückgeführt worden, bei dem der Antwortbolus von dem durchlaufenden Bolus getrennt ist. Die Bestimmung der Rezirkulation aus dem Antwortbolus erfolgt dann wie unter Bezugnahme auf Fig. 3 beschrieben ist.

Wenn die Dialysierflüssigkeitseingangskonzentration C_{di}(t) konstant gehalten wird und stattdessen die Pumpenförderrate variiert wird, kann die Rezirkulation entsprechend ermittelt werden. In diesem Fall wird die Pumpenförderrate in Abhängigkeit von der Zeit als Eingangsfunktion angesehen.

## Patentansprüche

1. Vorrichtung zur Bestimmung der Rezirkulation während einer extrakorporalen Blutbehandlung in Verbindung mit einer Blutbehandlungsvorrichtung, bei der das zu behandelnde Blut in einem extrakorporalen Kreislauf (7) die Blutkammer (10) eines durch eine semipermeable Membran (8a) in die Blutkammer (10) und eine Dialysierflüssigkeitskammer (9) unterteilten Dialysators (8) durchströmt und Dialysierflüssigkeit in einem Dialysierflüssigkeitsweg (11, 14) die Dialysierflüssigkeitskammer (9) des Dialysators (8) durchströmt, wobei in den extrakorporalen Kreislauf (7) eine Blutpumpe (25) und in den Dialysierflüssigkeitsweg eine Dialysierflüssigkeitspumpe (15) geschaltet ist, mit
einer Einrichtung (27) zum Einstellen der Förderrate der
Dialysierflüssigkeitspumpe oder der Blutpumpe,
einer Einrichtung (22,27) zum Verändern einer physikalischen oder chemischen Kenngröße des Blutes in einem vorgegebenen Zeitintervall, die eine Einrichtung (22) zum Verändern einer physikalischen oder chemischen Kenngröße der Dialysierflüssigkeit im Dialysierflüssigkeitsweg (11, 14) stromauf des Dialysators, die zu einer Veränderung der Kenngröße des Blutes führt, oder eine Einrichtung (27) zum Verändern der Förderrate der Blutpumpe (25) oder der Förderrate der Dialysierflüssigkeitspumpe (15) in einem vorgegebenen Zeitintervall ist, die zu einer Veränderung der Kenngröße des Blutes führt,
einer im Dialysierflüssigkeitsweg stromab des Dialysators (8) angeordneten Meßeinrichtung (17) zum Messen der auf die Veränderung der Kenngröße des Blutes zurückzuführenden Veränderung der Kenngröße der Dialysierflüssigkeit stromab des Dialysators,
einer Rechen- und Auswerteinheit (21), die zum Bestimmen der Rezirkulation aus dem zeitlichen Verlauf der Veränderung der physikalischen oder chemischen Kenngröße der Dialysierflüssigkeit stromab des Dialysators (8) ausgebildet ist.

2. Vorrichtung nach Anspruch 1, **dadurch gekennzeichnet, daß** eine Meßeinrichtung (13) zum Messen der Änderung der physikalischen oder chemischen Kenngröße der Dialysierflüssigkeit im Dialysierflüssigkeitsweg stromauf des Dialysators (8) vorgesehen ist.

3. Vorrichtung nach Anspruch 1 oder 2, **dadurch gekennzeichnet, daß** die Meßeinrichtung (17 bzw. 13) zum Messen der physikalischen oder chemischen Kenngröße der Dialysierflüssigkeit stromab bzw. stromauf des Dialysators (8) eine Meßeinrichtung zur Bestimmung der Dialysierflüssigkeits-Ionen-Konzentration oder der Temperatur der Dialysierflüssigkeit als physikalische oder chemische Kenngröße ist.

4. Vorrichtung nach Anspruch 3, **dadurch gekennzeichnet, daß** die Meßeinrichtung (17 bzw. 13) einen Leitfähigkeitssensor oder einen optischen Sensor zur Bestimmung der Dialysierflüssigkeits-Ionen-Konzentration bzw. einen Temperatursensor zur Bestimmung der Temperatur der Dialysierflüssigkeit aufweist.

## Claims

1. A device for monitoring recirculation during an extracorporeal blood treatment activity in connection with a blood treatment device in which the blood to be treated flows in an extracorporeal circuit (7) through the blood chamber (10) of a dialyser (8) subdivided by a semi-permeable membrane (8a) into a blood chamber (10) and a dialysis liquid chamber (9) and the dialysis liquid flows in a dialysis liquid path (11, 14) through the dialysis liquid chamber (9) of the dialyser (8) whereby a blood pump (25) is included in the extracorporeal circuit (7) and a dialysis liquid pump (15) is included in the dialysis liquid pathway, with
a device (27) for adjusting the delivery rate of either the dialysis liquid pump and the blood pump,
a device (22, 27) for modifying a physical or chemical characteristic of the blood over a predetermined time interval which is a device (22) for modifying a physical or chemical characteristic of the dialysis liquid in the dialysis liquid flowpath (11, 14) which is located upstream of the dialyser which leads to a modification of a characteristic of the blood,
or a device (27) for modifying the delivery rate of the blood pump (25) or the delivery rate of the dialysis liquid pump (15) over a predetermined time interval which leads to a modification of the characteristic of the blood,
a measuring device (17) arranged in the dialyser liquid flowpath downstream of the dialyser (8) to measure the change in the characteristic of the dialysis liquid brought about by the modification of the characteristic of the blood downstreams of the dialyser,
a calculating- and evaluation unit (21) which is designed to monitor the recirculation from the period of time associated with the modification of the physical or chemical characteristic of the dialysis liquid downstream of the dialyser (8).

2. Device in accordance with claim 1, **characterised in that** a measuring device (13) is provided for measuring the change in the physical or chemical characteristic of the dialysis liquid in the dialysis liquid flowpath upstream of the dialyser (8).

3. Device in accordance with claim 1 or 2, **characterised in that** the measuring device (17 or 13) for measuring the physical or chemical characteristic of the dialysis liquid upstream or downstream of the dialyser (8) is a measuring device for determining the ion-concentration of the dialysis liquid or the temperature of the dialysis liquid as a physical or chemical characteristic.

4. Device in accordance with claim 3, **characterised in that** the measuring device (17 or 13) possesses a conductive sensor or an optical sensor for the determination of the ion-concentration in the dialysis liquid or a temperature sensor for determining the temperature of the dialysis liquid.

## Revendications

1. Dispositif destiné à déterminer la recirculation pendant un traitement extracorporel du sang en liaison avec un dispositif de traitement du sang, dans lequel le sang à traiter circule dans un circuit extracorporel (7) à travers le compartiment à sang (10) d'un dialyseur (8), qui est séparé par une membrane (8a) semi-perméable en un compartiment à sang (10) et un compartiment à liquide de dialyse (9), et le liquide de dialyse circule dans un trajet pour liquide de dialyse (11, 14) à travers le compartiment à liquide de dialyse (9), une pompe pour le sang (25) étant montée dans le circuit extracorporel (7) et une pompe pour le liquide de dialyse (15) étant montée dans le trajet pour liquide de dialyse (11, 14), comprenant
une unité (27) destinée à régler la vitesse de refoulement de la pompe pour le liquide de dialyse ou de la pompe pour le sang, une unité (22, 27) destinée à faire varier un paramètre caractéristique physique ou chimique du sang dans un intervalle de temps prédéfini, qui est soit une unité (22) destinée à faire varier le paramètre caractéristique physique ou chimique du liquide de dialyse dans le trajet pour liquide de dialyse (11, 14) en amont du dialyseur, ce qui entraîne une variation du paramètre caractéristique du sang, soit une unité (27) destinée à faire varier la vitesse de refoulement de la pompe pour le sang (25) ou la vitesse de refoulement de la pompe pour le liquide de dialyse (15) dans un intervalle de temps prédéfini, ce qui entraîne une variation du paramètre caractéristique du sang,
une unité de mesure (17), disposée dans le trajet pour le liquide de dialyse en aval du dialyseur (8) et destinée à mesurer la variation du paramètre caractéristique du liquide de dialyse en aval du dialyseur, laquelle variation est due à la variation du paramètre caractéristique du sang,
une unité de calcul et d'analyse (21), qui est conçue pour déterminer la recirculation à partir de l'évolution en fonction du temps de la variation du paramètre caractéristique physique ou chimique du liquide de dialyse en aval du dialyseur (8).

2. Dispositif selon la revendication 1, **caractérisé en ce que** une unité de mesure (13), destinée à mesurer la variation du paramètre caractéristique physique ou chimique du liquide de dialyse, est prévue dans le trajet pour le liquide de dialyse en amont du dialyseur (8).

3. Dispositif selon la revendication 1 ou 2, **caractérisé en ce que** l'unité de mesure (17 ou 13), destinée à mesurer la variation du paramètre caractéristique physique ou chimique du liquide de dialyse respectivement en aval ou en amont du dialyseur (8), est une unité de mesure destinée à déterminer la concentration en ions dans le liquide de dialyse ou la température du liquide de dialyse en tant que paramètre caractéristique physique ou chimique.

4. Dispositif selon la revendication 3, **caractérisé en ce que** l'unité de mesure (17 ou 13) comporte un capteur de conductibilité ou un capteur optique pour déterminer la concentration en ions dans le liquide de dialyse ou un capteur de température pour déterminer la température du liquide de dialyse.
